# EUROPÄISCHE PATENTSCHRIFT

(11) **EP 2 255 949 B1**
(45) Veröffentlichungstag und Bekanntmachung des Hinweises auf die Patenterteilung: **05.10.2011**
(21) Anmeldenummer: 10161461.8
(22) Anmeldetag: 29.04.2010
(51) Int. Cl.: B29C 49/42, B29C 49/58, B29C 49/36

(54) **Blasmaschine mit CIP-Reinigungssystem zur Herstellung von Kunststoff-Flaschen, und entsprechendes Verfahren**
Blowing machine with CIP cleaning system for producing plastic bottles, and the corresponding method
Souffleuse dotée d'un système de nettoyage CIP pour la fabrication de bouteilles en plastique, et procédé de réalisation correspondant

(30) Priorität: 29.05.2009 DE 102009023406
(43) Veröffentlichungstag der Anmeldung: 01.12.2010
(73) Patentinhaber: Krones AG, 93073 Neutraubling (DE)
(72) Erfinder: Voth, Klaus, 93083, Obertraubling (DE)
(74) Vertreter: Grünecker, Kinkeldey, Stockmair & Schwanhäusser Anwaltssozietät

(56) Entgegenhaltungen:
- WO-A1-02/092324
- DE-U1- 20 023 423
- US-A- 3 340 569

## Beschreibung

Die Erfindung betrifft eine Blasmaschine zur Herstellung von Kunststoff-, insbesondere PET-Flaschen nach dem Oberbegriff des Anspruchs 1, sowie ein Verfahren zur CIP-Reinigung einer Blasmaschine.

Das sogenannte "Cleaning-In-Place", auch bezeichnet als CIP-Reinigung, ist ein bekanntes Verfahren zur Entkeimung von Produktionsanlagen in der Lebensmittelindustrie, bei dem auf eine Demontage der vom Produkt bzw. den Zwischenprodukten und Hilfsstoffen berührten Flächen im Wesentlichen verzichtet werden kann.

Die DE 200 23 423 U1 beschreibt eine Vorrichtung zum Herstellen von Behältern durch Formblasen, bei der eine Blasform mit mindestens einem Einlass für ein Beschichtungsmittel vorgesehen ist.

Die WO 02/092324 A1 beschreibt eine Vorrichtung zum Einspritzen einer Reinigungsflüssigkeit in einen Kunststoffvorformling, wobei dieser von einem Gehäuse aufgenommen wird und die Reinigungsflüssigkeit mit Hilfe einer Kanüle durch eine in dem Gehäuse vorgesehene Bohrung zugeführt wird.

Die US 3,340,569 A beschreibt eine Blasmaschine zum Herstellen von Kunststoffgefäßen, wobei die geblasenen Gefäße vor dem Aushärten aus den Blasformen entnommen werden können. An der Vorrichtung sind ferner Ventile bestehend aus zwei miteinander verschraubten Hälften vorgesehen, die für die Inspektion, die Reinigung oder den Austausch geöffnet und entfernt werden können.

Die CIP-Entkeimung von Blasmaschinen für Getränkeflaschen, wie die der DE 200 18 500 U1, ist jedoch bisher nicht möglich, da die üblicherweise zur Entkeimung verwendeten Reinigungsmittel, wie z. B. Wasserstoffperoxid oder Peressigsäure, die Pneumatikeinheiten solcher Blasmaschinen angreifen.

Die stattdessen erforderliche Reinigung und Entkeimung mit Teil-Demontage der Blasmaschine ist zeitaufwändig und verursacht einen unerwünscht langen Stillstand der Maschine. Es ist daher Aufgabe der Erfindung, eine Blasmaschine bereitzustellen, die sich einfacher und schneller entkeimen lässt. Ebenso benötigt wird ein Verfahren zu deren Reinigung. Die Aufgabe wird dadurch gelöst, dass die Ventilblöcke der Blasmaschine so ausgebildet sind, dass sie in die CIP-Reinigung mit einbezogen werden können.

Es ist dadurch möglich, die für die Herstellung von PET-Flaschen aus hygienischer Sicht besonders kritischen Anlagenteile, wie die Blasdüse, im Wesentlichen ohne Demontage zu reinigen und zu entkeimen.

Vorzugsweise sind die Ventile pneumatisch zu betätigen. Die Blasmaschine kann damit zuverlässig und kostengünstig gesteuert werden.

Bei einer besonders vorteilhaften Weiterbildung der Erfindung sind an den Ventilen CIP-Absperrventile zum Absperren pneumatischer Steuerleitungen der Steuerventile vorgesehen. Auf diese Weise kann verhindert werden, dass aggressive CIP-Reinigungsmittel durch die Steuerleitung an weitere Steuerventile und/oder in ein Druckluftnetz gelangen können. Vorzugsweise umfassen die Ventile Dichtungen, die beständig gegen entkeimende CIP-Reinigungsmittel sind, insbesondere gegen Wasserstoffperoxid, Peressigsäure, Alkohol und Seifenlauge. Dies ermöglicht eine komplikationslose, wiederholte Reinigung der Ventile.

Steuerventile werden herkömmlich auch als Pilotventile bezeichnet.

Bei einer besonders vorteilhaften Ausführungsform umfasst das CIP-Reinigungssystem: eine Aufbereitungseinheit zum Umwälzen und Aufbereiten eines CIP-Reinigungsmittels; eine CIP-Zuleitung für das Reinigungsmittel, die mit einer Blasluft-Zuleitung für die Steuerventile verbunden werden kann; und abnehmbare CIP-Dichtkappen zum Auffangen des Reinigungsmittels an den Blasstationen, wobei die CIP-Dichtkappen mit einer CIP-Rücklaufleitung zum Zurückführen des Reinigungsmittels in die Aufbereitungseinheit verbunden sind. Dadurch kann das Reinigungsmittel vollständig gesammelt und im Kreislauf gefahren werden.

Vorzugsweise umfasst die Blasmaschine ferner einen Medienverteiler zum Verteilen der Blasluft auf die Blasstationen und zum Sammeln und Einleiten des aufgefangenen Reinigungsmittels in die CIP-Rücklaufleitung. Dadurch kann die Blasluft und das Reinigungsmittel platzsparend verteilt werden und die Blasmaschine schnell von Produktionsbetrieb auf CIP-Reinigung und umgekehrt umgestellt werden.

Vorzugsweise ist in der CIP-Zuleitung ein CIP-Hauptabsperrventil vorgesehen. Dieses verhindert im Produktionsbetrieb ein Eindringen von Blasluft in die Aufbereitungseinheit und/oder das Eindringen von Reinigungsmittel in den Blasluftstrom.

Vorzugsweise ist in der Blasluft-Zuleitung ein Blasluft-Absperrventil vorgesehen. Dieses verhindert bei der CIP-Reinigung das Eindringen von Reinigungsmittel in einen Blasluftkompressor und/oder das Austreten von Blasluft in den CIP-Kreislauf.

Die Problemstellung wird ebenso mit einem Verfahren zur CI P-Reinigung der erfindungsgemäßen Blasmaschine gelöst, bei dem in einem Schritt a) ein CIP-Reinigungsmittel durch die Ventilblöcke der Blasstationen geleitet wird, einschließlich der Ventile und der Blasdüsen. Für die Herstellung von PET-Flaschen aus hygienischer Sicht besonders kritische Anlagenteile der Blasmaschine, wie die Blasdüse, sind so ohne Demontage zu reinigen und zu entkeimen.

Vorzugsweise umfasst das CIP-Reinigungsverfahren ferner folgenden Schritt b): Absperren pneumatischer Steuerleitungen der Steuerventile, wobei Schritt b) vor Schritt a) ausgeführt wird. Aggressive CIP-Reinigungsmittel können somit nicht durch die Steuerleitung an weitere Steuerventile und/oder in ein Druckluftnetz gelangen.

Bei einer besonders günstigen Ausgestaltung umfasst das CIP-Reinigungsverfahren ferner folgenden Schritt c): Verbinden einer CIP-Zuleitung für das Reinigungsmittel mit mindestens einer Blasluft-Zuleitung zu den Ventilen, wobei Schritt c) vor Schritt a) ausgeführt wird. Dadurch können die vorhandenen Blasluftkanäle gereinigt und für die Einleitung des Reinigungsmittels in die Blasdüse genutzt werden.

Vorzugsweise umfasst das Verfahren ferner folgenden Schritt d): Absperren eines kompressorseitigen Abschnitts der Blasluft-Zuleitung, wobei Schritt d) vor Schritt a) ausgeführt wird. Dies verhindert bei der CIP-Reinigung das Eindringen von Reinigungsmittel in einen Blasluftkompressor und/oder das Austreten von Blasluft in den CIP-Kreislauf.

Vorzugsweise umfasst das Verfahren ferner folgenden Schritt: Auffangen des durch die Ventilblöcke geleiteten Reinigungsmittels und Rückführen des Reinigungsmittels in einen CIP-Kreislauf. Dadurch wird verhindert, dass Reinigungsmittel unkontrolliert austritt bzw. verschwendet wird.

Eine besonders günstige Ausgestaltung des Verfahrens umfasst ferner folgenden Schritt: Aufbereiten des zurückgeführten Reinigungsmittels und Einspeisen des aufbereiteten Reinigungsmittels in den CIP-Kreislauf. Dadurch kann das Reinigungsmittel bei gleichbleibender Reinigungsqualität wiederholt verwendet werden. Dies spart Kosten und schont die Umwelt.

Vorzugsweise wird ein entkeimendes Reinigungsmittel durch die Ventilblöcke geleitet, insbesondere Wasserstoffperoxid, Peressigsäure, Alkohol oder Seifenlauge. Damit kann die Entkeimung in für Getränkeanlagen bewährter Weise erfolgen, insbesondere mit Reinigungsmittel, wie sie auch für die Entkeimung von Preforms verwendet werden.

Eine bevorzugte Ausführungsform der erfindungsgemäßen Blasmaschine ist in der Zeichnung dargestellt. Es zeigen:
- Fig. 1: eine schematische Darstellung einer erfindungsgemäßen Blasmaschine mit CIP- Reinigungssystem;
- Fig.2: einen schematischen Querschnitt durch einen Ventilblock mit einem pneumatisch gesteuerten Ventil zum Einleiten von Blasluft.

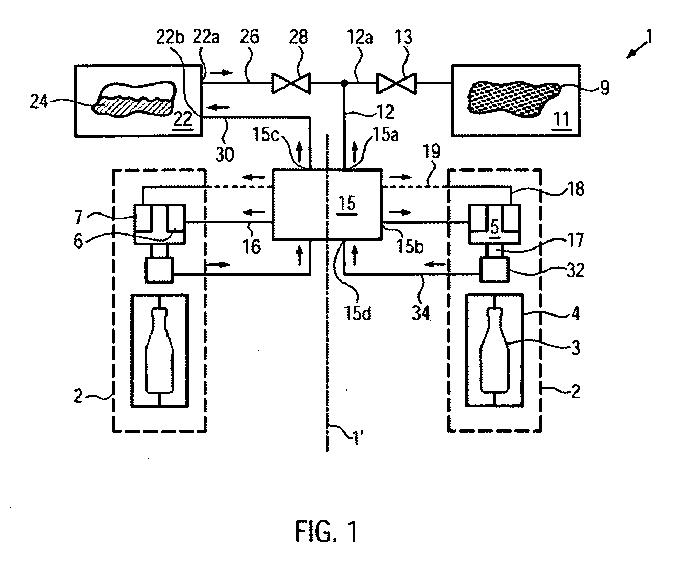

Wie Fig. 1 erkennen lässt, ist die Blasmaschine 1 im Ausführungsbeispiel in bekannter Weise als Rundläufer mit mehreren, um eine Drehachse 1' symmetrisch angeordneten Blasstationen 2 zum Herstellen von Kunststoff-Flaschen 3 ausgebildet, wobei an jeder Blasstation 2 eine Blasform 4 und ein Ventilblock 5 mit Ventilen 6,7 zum Einleiten bzw. Ausleiten von Blasluft 9 vorgesehen ist.

Ein Kompressor 11 zum Erzeugen der Blasluft 9 ist durch eine Blasluft-Zuleitung 12 über ein Blasluft-Absperrventil 13 mit dem Blasluft-Eingang 15a eines Medienverteilers 15 zur Verteilung der Blasluft 9 auf die einzelnen Blasstationen 2 verbunden. Die Blasluft-Ausgänge 15b des Medienverteilers 15 sind durch Blasluft-Zuleitungskanäle 16 jeweils mit einem Steuerventil 6 zum Einleiten der Blasluft 9 in eine im Ventilblock 5 vorgesehene Blasdüse 17 verbunden. An dem Ventilblock 5 bzw. der Blasstation 2 ist außerdem mindestens ein Ventil 7 und ein Blasluft-Ausleitkanal 18 zum Ausleiten der Blasluft 9 aus der Blasdüse 17 bzw. der Blasform 4 vorgesehen. Die Fig. 1 zeigt die Blasmaschine 1 in einer Stellung mit gegenüber der Blasform 4 angehobenem Ventilblock 5, so dass die Blasdüse 17 von unten zugänglich ist.

Die Blasmaschine 1 umfasst ferner ein CIP-Reinigungssystem 20 mit: einer Aufbereitungseinheit 22 zum Umwälzen und Aufbereiten eines Reinigungsmittels 24; einer CIP-Zuleitung 26, die einen Ausgang 22a der Aufbereitungseinheit 22 über ein CIP-Hauptabsperrventil 28 mit der Blasluft-Zuleitung 12 bzw. dem Blasluft-Eingang 15a des Medienverteilers 15 verbindet; einer CIP-Rücklaufleitung 30, die einen CIP-Ausgang 15c des Medienverteilers 15 mit einem Eingang 22b der Aufbereitungseinheit 22 verbindet; und abnehmbaren CIP-Dichtkappen 32 mit CIP-Auffangleitungen 34 zum Auffangen und Zurückleiten des Reinigungsmittels 24 aus den Blasdüsen 17 in die CIP-Eingänge 15d des Medienverteilers 15.

Im CIP-Reinigungsbetrieb pumpt die Aufbereitungseinheit 22 das Reinigungsmittel 24 bei geschlossenem Blasluft-Absperrventil 13 und geöffnetem CIP-Hauptabsperrventil 28 durch die CIP-Zuleitung 26, die Blasluft-Zuleitung 12, den Medienverteiler 15, die Blasluft-Zuleitungskanäle 16, die Ventile 6, die Blasdüsen 17, die CIP-Dichtkappen 32, die CIP-Auffangleitungen 34, den Medienverteiler 15 und die CIP-Rücklaufleitung 30 im Kreislauf. Die Fließrichtung des Reinigungsmittels 24 ist in Figur 1 durch Pfeile symbolisiert. Das Blasluft-Absperrventil 13 verschließt nur den kompressorseitigen Abschnitt 12a der Blasluft-Zuleitung 12, so dass weder Blasluft 9 in den CIP-Kreislauf noch Reinigungsflüssigkeit 24 zum Kompressor 11 gelangt.

Im CIP-Reinigungsbetrieb wird das Reinigungsmittel 24 gleichzeitig durch die Blasluft-Ausleitkanäle 18 und die Ventile 7 in die Blasdüsen 17 gepumpt. Da die Ausleitkanäle 18 im Produktionsbetrieb normalerweise in die umgebende Raumluft führen, sind diese während der CIP-Reinigung über (gestrichelt gezeichnete) CIP-Verbindungsleitungen 19 mit der CIP-Zuleitung 26 bzw. dem Medienverteiler 15 oder der Blasluft-Zuleitung 12 verbunden.

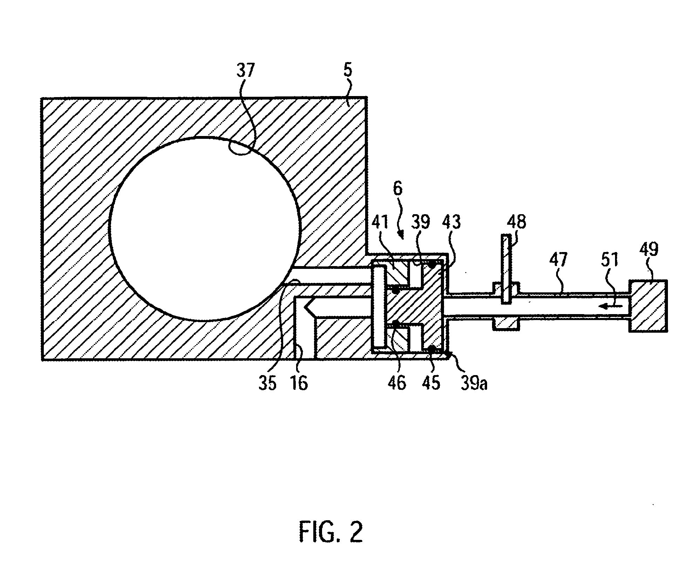

Fig. 2 zeigt den Ventilblock 5 im Querschnitt, vereinfacht mit nur einem pneumatisch betätigten, geöffneten Ventil 6 zur Einleitung der Blasluft 9 durch den Blasluft-Zuleitungskanal 16 und einen Verbindungskanal 35 in die Blasdüse 17, für die im Ventilblock 5 eine Aufnahmebohrung 37 vorgesehen ist. Das Ventil 6 umfasst einen Zylinder 39 mit einer Distanzbuchse 41, und einen Kolben 43 mit Dichtungen 45, 46, die den Kolben 43 gegen den Zylinder 39 bzw. die Distanzbuchse 41 abdichten.

Das Ventil 6 ist über eine pneumatische Steuerleitung 47, ein CIP-Absperrventil 48 (in Fig.1 der Übersichtlichkeit halber jeweils nicht gezeigt) und ein herkömmliches Steuer- bzw. Pilotventil 49 zum Öffnen/Schließen des Ventils 6 mit einem Druckluftnetz verbunden, das Druckluft 51 von beispielsweise 6 bar liefert. Im Produktionsbetrieb ist das CIP-Absperrventil 48 geöffnet. Bei Öffnen des Pilotventils 49 drückt die Druckluft 51 den Kolben 43 in Richtung des Blasluft-Zuleitungskanals 16, bis dieser verschlossen ist. Nach Schließen des Pilotventils 49 kann das Ventil 6 durch die über den Blasluft-Zuleitungskanal 16 einströmende und gegen den Kolben 43 drückende Blasluft 9 wieder geöffnet werden.

Im CIP-Reinigungsbetrieb ist das CIP-Absperrventil 48 geschlossen, um zu verhindern, dass das Reinigungsmittel 24 in das Pilotventil 49 gelangt. Da die Druckluft 51 am Ventil 6 dann nicht anliegt, drückt das durch den Blasluft-Zuleitungskanal 16 einströmende Reinigungsmittel 24 den Kolben 43 nach rechts, öffnet somit das Ventil 6, und gelangt durch den Verbindungskanal 35 in die Blasdüse 17.

Die Dichtungen 45, 46 verhindern das Eindringen des Reinigungsmittels 24 in den ansteuerungsseitigen Bereich 39a zwischen Kolben 43 und Zylinder 39 vorzugsweise vollständig. Es ist jedoch auch denkbar, dass in den Bereich 39a eingedrungenes Reinigungsmittel 24 zum Abschluss der CIP-Reinigung nach Öffnen des CIP-Absperrventils 48 durch die Druckluft 51 aus dem Zylinder 39 in die Blasdüse 17 geblasen wird. Ebenso wäre es möglich, den Bereich 39a über einen (nicht dargestellten) im Ventilblock 5 ausgebildeten Entlüftungskanal zu entleeren, gegebenenfalls unterstützt durch eine Erwärmung des Bereichs 39a.

Die Dichtungen 45, 46 bestehen aus einem gegen das Reinigungsmittel 24 beständigen Material, wie z. B. Metall, EPDM-Kautschuk, Perfluorkautschuk (z.B. FFKM) oder einem geeigneten Materialverbund. Bevorzugt können auch Teflonringe verwendet werden. Die gegen die Distanzbuchse 41 abdichtende Dichtung 46 ist nicht zwingend notwendig.

Das CIP-Absperrventil 48 kann z. B. ein elektromagnetisch betätigtes Absperrventil sein, beispielsweise ein Hahn oder Riegel.

Das Reinigungsmittel 24 ist bevorzugt ein CIP-Entkeimungsmittel, wie es beispielsweise zur Entkeimung von Preforms verwendet wird. Das Reinigungsmittel 24 ist vorzugsweise eine Säure oder Lauge, wie z. B. Peressigsäre, Wasserstoffperoxid oder Seifenlauge. Auch die Verwendung von Alkoholen ist denkbar.

Die abnehmbare CIP-Dichtkappe 32 schließt mit dem Ventilblock 5 bzw. der Blasdüse 17 dicht ab, so dass das zirkulierende Reinigungsmittels 24 möglichst vollständig aufgefangen, zurückgeführt und aufbereitet werden kann. Die CIP-Auffangleitung 34 kann z. B. eine flexible Leitung sein. Entscheidend ist, dass die CIP-Dichtkappe 32 für die Reinigung aufgesetzt bzw. für den Produktionsbetrieb abgenommen werden kann.

Blasmaschinen arbeiten üblicherweise mit Blasluft 9 unterschiedlicher Druckbereiche, z. B. zum Vorblasen, Fertigblasen und Zwischenblasen, so dass dann mehrere Ventile 6 zum Einleiten der Blasluft 9 vorgesehen sind. Diese werden in der Regel abwechselnd geöffnet. Ebenso sind gegebenenfalls mehrere Entlastungsventile 7 zum Ausleiten der Blasluft 9 vorgesehen, die in der Regel gemeinsam geöffnet werden. In Fig. 1 ist der Übersichtlichkeit halber nur ein Drucksystem, z. B. das für die Fertigblasluft, gezeigt. Es versteht sich von selbst, dass in der erfindungsgemäßen Blasmaschine 1 zusätzliche Ventile 6,7 und zugehörige Leitungen für zusätzliche Nieder- bzw. Hochdrucksysteme vorgesehen werden können.

Ebenso stellt die Fig. 2 vereinfachend nur ein Ventil 6 und die zugehörigen Kanäle 16, 35 dar. An dem Ventilblock 5 kann aber eine beliebige Anzahl von Ventilen 6,7 mit den zugehörigen Kanälen 16, 35 vorgesehen sein. Ebenso kann die Bauweise vom gezeigten Beispiel abweichen. Die Entlastungsventile 7 können nach dem gleichen Funktionsprinzip wie die einleitenden Ventilen 6 ausgeführt werden. Es versteht sich jedoch von selbst, dass die Lage des Verbindungskanals 35 und des Ausleitkanals 18 von der Fig. 2 abweichen können. Beispielsweise könnte der Kolben 43 genauso gut den Verbindungskanal 35 verschließen. Entscheidend ist, dass die Ventile 6, 7, und insbesondere die Dichtungen 45, 46, vom Reinigungsmittel 24 nicht angegriffen werden und so in das CIP-Reinigungssystem 20 integriert werden können.

Das CIP-Reinigungssystem 20 arbeitet vorzugsweise im Kreislauf mit Aufbereitung des Reinigungsmittels 24. Dadurch lässt sich das Reinigungsmittel wiederholt mit gleichbleibender Entkeimungswirkung einsetzen, um Ressourcen zu sparen. Das Reinigungsmittel 24 könnte nach Durchströmen der Blasdüse 17 jedoch auch von der CIP-Dichtkappe 32 aufgefangen und einem Tank zur Entsorgung oder separaten Aufarbeitung zugeführt werden.

Die erfindungsgemäße Blasmaschine 1 kann wie folgt gereinigt werden:

Das Blasluft-Absperrventil 13 und die CIP-Absperrventile 48 werden geschlossen. Nach Aufsetzen der CIP-Schutzkappen 32 auf die Blasdüsen 17 und Verbinden der Blasluft-Ausleitkanäle 18 mit den Ausgängen 15b des Medienverteilers 15 bzw. der Blasluft-Zuleitung 12 wird das CIP-Hauptabsperrventil 28 geöffnet und die Aufbereitungseinheit 22 gestartet. Diese pumpt Reinigungsmittel 24 durch die Blasluft-Zuleitungskanäle 16 und die Blasluft-Ausleitungskanäle 18 bis an die Kolben 43 der Ventile 6,7. Durch den Druck des Reinigungsmittels 24 werden die Kolben 43 verschoben und somit die Ventile 6,7 geöffnet, so dass das Reinigungsmittel 24 durch die Verbindungskanäle 35 in die Blasdüsen 17 strömt. Dabei verhindern die Dichtungen 45,46 und die CIP-Absperrventile 48 ein Vordringen des Reinigungsmittels 24 bis zu den Pilotventilen 49. Das Reinigungsmittel 24 wird von den CIP-Dichtkappen 32 aufgefangen und durch den Medienverteiler 15 und die Rückführleitung 30 in die Aufbereitungseinheit 22 zurückgeleitet. Diese regeneriert das Reinigungsmittel 24 und pumpt es wieder in den CIP-Kreislauf. Nach Erreichen einer vorgegebenen CIP-Spülmenge bzw. -dauer wird die Pumpfunktion der Aufbereitungseinheit 22 beendet und das CIP-Hauptabsperrventil 28 wieder geschlossen. Restliches Reinigungsmittel 24 kann nun in die CIP-Dichtkappe 32 abgelassen werden und/oder in diese, nach Öffnen des Blasluft-Absperrventils 13, mit Blasluft 9 geblasen werden. Zusätzlich kann nach Öffnen des CIP-Absperrventils 48 bei Bedarf an den Dichtungen 45,46 übergetretenes Reinigungsmittel 24 mit der Steuer-Druckluft 51 aus dem Ventilzylinder 39 in die Blasdüse 17 geblasen werden. Das beim Entleeren der Zuleitungen 12,16,18 und des Medienverteilers 15 aufgefangene Reinigungsmittel 24 wird ebenfalls in den CIP-Kreislauf zurückgeführt oder entsorgt.

## Patentansprüche

1. Blasmaschine (1) zur Herstellung von Kunststoff-, insbesondere PET-Flaschen (3), mit:
- mehreren Blasstationen (2), wobei diesen Ventilblöcke (5) mit Ventilen (6,7) und Blasdüsen (17) zum Ein- bzw. Ausleiten von Blasluft (9) zugeordnet sind; und
- einem Cleaning-In-Place (CIP)-Reinigungssystem (20) zum Reinigen der Blasmaschine (1),
**dadurch gekennzeichnet, dass**
die Ventilblöcke (5) so ausgebildet sind, dass sie in die CIP-Reinigung mit einbezogen werden können.

2. Blasmaschine nach Anspruch 1, **dadurch gekennzeichnet, dass** die Ventile (6,7) pneumatisch zu betätigen sind.

3. Blasmaschine nach mindestens einem der vorigen Ansprüche, **dadurch gekennzeichnet, dass** an den Ventilen (6,7) CIP-Absperrventile (48) zum Absperren pneumatischer Steuerleitungen (47) der Ventile (6,7) vorgesehen sind.

4. Blasmaschine nach mindestens einem der vorigen Ansprüche, **dadurch gekennzeichnet, dass** die Ventile (6,7) Dichtungen (45,46) umfassen, die beständig gegen entkeimende CIP-Reinigungsmittel sind, insbesondere gegen Wasserstoffperoxid, Peressigsäure, Alkohol und Seifenlauge.

5. Blasmaschine nach mindestens einem der vorigen Ansprüche, **dadurch gekennzeichnet, dass** das CIP-Reinigungssystem (20) umfasst:
- eine Aufbereitungseinheit (22) zum Umwälzen und Aufbereiten eines CIP-Reinigungsmittels (24);
- eine CIP-Zuleitung (26) für das Reinigungsmittel (24), die mit einer Blasluft-Zuleitung (12) für die Ventile (6,7) verbunden werden kann; und
- aufsetzbare/abnehmbare CIP-Dichtkappen (32) zum Auffangen des Reinigungsmittels (24) an den Blasstationen (2), wobei die CIP-Dichtkappen (32) mit einer CIP-Rücklaufleitung (30) zum Zurückführen des Reinigungsmittels (24) in die Aufbereitungseinheit (22) verbunden sind.

6. Blasmaschine nach Anspruch 5, ferner **gekennzeichnet durch** einen Medienverteiler (15) zum Verteilen der Blasluft (9) auf die Blasstationen (2) und zum Sammeln und Einleiten des aufgefangenen Reinigungsmittels (24) in die CIP-Rücklaufleitung (30).

7. Blasmaschine nach Anspruch 5 oder 6, **dadurch gekennzeichnet, dass** in der CIP-Zuleitung (26) ein CIP-Hauptabsperrventil (28) vorgesehen ist.

8. Blasmaschine nach mindestens einem der Ansprüche 5 bis 7, **dadurch gekennzeichnet, dass** in der Blasluft-Zuleitung (12) ein Blasluft-Absperrventil (13) vorgesehen ist.

9. Verfahren zur CIP-Reinigung der Blasmaschine (1) nach Anspruch 1, folgenden Schritt umfassend:
a) Durchleiten eines CIP-Reinigungsmittels (24) durch die Ventilblöcke (5) der Blasstationen (2), einschließlich der Ventile (6,7) und der Blasdüsen (17).

10. Verfahren nach Anspruch 9, ferner folgenden Schritt umfassend:
b) Absperren pneumatischer Steuerleitungen (47) der Ventile (6,7), wobei Schritt b) vor Schritt a) ausgeführt wird.

11. Verfahren nach Anspruch 9 oder 10, ferner folgenden Schritt umfassend:
c) Verbinden einer CIP-Zuleitung (26) für das Reinigungsmittel (24) mit mindestens einer Blasluft-Zuleitung (12) für die Ventile (6,7), wobei Schritt c) vor Schritt a) ausgeführt wird.

12. Verfahren nach Anspruch 11, ferner folgenden Schritt umfassend:
d) Absperren eines kompressorseitigen Abschnitts (12a) der Blasluft-Zuleitung (12), wobei Schritt d) vor Schritt a) ausgeführt wird.

13. Verfahren nach einem der Ansprüche 9 bis 12, ferner folgenden Schritt umfassend:
e) Auffangen des durch die Ventilblöcke (5) geleiteten Reinigungsmittels (24) und Rückführen des Reinigungsmittels (24) in einen CIP-Reinigungsmittelkreislauf.

14. Verfahren nach Anspruche 13, ferner folgenden Schritt umfassend:
f) Aufbereiten des zurückgeführten Reinigungsmittels (24) und Einspeisen des aufbereiteten Reinigungsmittels (24) in den CIP-Reinigungsmittelkreislauf.

15. Verfahren nach einem der Ansprüche 9 bis 14, wobei ein entkeimendes Reinigungsmittel durch die Ventilblöcke (5) geleitet wird, insbesondere Wasserstoffperoxid, Peressigsäure, Alkohol oder Seifenlauge.

## Claims

1. Blowing machine (1) for producing plastic, in particular PET bottles (3), comprising:
- several blowing stations (2) to which valve blocks (5) with valves (6, 7) and blowing nozzles (17) for introducing and discharging blowing air (9) are assigned; and
a cleaning-in-place cleaning system (20) (CIP) for cleaning the blowing machine (1), **characterised in that**
the valve blocks (5) are designed so that they can be involved in the CIP cleaning process.

2. Blowing machine as claimed in claim 1, **characterised in that** the valves (6, 7) are pneumatically operated.

3. Blowing machine as claimed in at least one of the preceding claims, **characterised in that** CIP shut-off valves (48) are provided on the valves (6, 7) for shutting off pneumatic control lines (47) of the valves (6, 7).

4. Blowing machine as claimed in at least one of the preceding claims, **characterised in that** the valves (6, 7) have seals (45, 46) which are resistant to sterilising CIP cleaning agents, in particular to hydrogen peroxide, peracetic acid, alcohol and soap suds.

5. Blowing machine as claimed in at least one of the preceding claims, **characterised in that** the CIP cleaning system (20) comprises:
- a processing unit (22) for circulating and processing a CIP cleaning agent (24);
- a CIP feed line (26) for the cleaning agent (24), which can be connected to a blowing air feed line (12) for the valves (6, 7); and
- attachable/detachable CIP sealing caps (32) for collecting the cleaning agent (24) at the blowing stations (2), which CIP sealing caps (32) are connected to a CIP return line (30) for returning the cleaning agent (24) to the processing unit (22).

6. Blowing machine as claimed in claim 5, further **characterised by** a medium distributor (15) for distributing the blowing air (9) to the blowing stations (2) and for collecting and introducing the collected cleaning agent (24) into the CIP return line (30).

7. Blowing machine as claimed in claim 5 or 6, **characterised in that** a CIP main shut-off valve (28) is provided in the CIP feed line (26).

8. Blowing machine as claimed in at least one of claims 5 to 7, **characterised in that** a blowing air shut off valve (13) is provided in the blowing air feed line (12).

9. Method of CIP cleaning the blowing machine (1) claimed in claim 1, comprising the following step:
a) circulating a CIP cleaning agent (24) through the valve blocks (5) of the blowing stations (2), including the valves (6, 7) and blowing nozzles (17).

10. Method as claimed in claim 9, further comprising the following step:
b) shutting off pneumatic control lines (47) of the valves (6, 7), step b) being carried out prior to step a).

11. Method as claimed in claim 9 or 10, further comprising the following step:
c) connecting a CIP feed line (26) for the cleaning agent (24) to at least one blowing air feed line (12) for the valves (6, 7), step c) being carried out prior to step a).

12. Method as claimed in claim 11, further comprising the following step:
d) shutting off a compressor-end portion (12a) of the blowing air feed line (12), step d) being carried out prior to step a).

13. Method as claimed in one of claims 9 to 12, further comprising the following step:
e) collecting the cleaning agent (24) directed through the valve blocks (5) and returning the cleaning agent (24) to a CIP cleaning agent circuit.

14. Method as claimed in claim 13, further comprising the following step:
f) processing the returned cleaning agent (24) and feeding the processed cleaning agent (24) into the CIP cleaning agent circuit.

15. Method as claimed in one of claims 9 to 14, whereby a sterilising cleaning agent is fed through the valve blocks (5), in particular hydrogen peroxide, peracetic acid, alcohol or soap suds.

## Revendications

1. Souffleuse (1) pour la fabrication de bouteilles (3) en matière plastique, en particulier en PET, avec :
- plusieurs stations de soufflage (2), étant précisé que des blocs de commande (5) avec des soupapes (6, 7) et des buses de soufflage (17) pour amener et évacuer de l'air de soufflage (9) sont associés à ces stations de soufflage (2) ; et
- un système de nettoyage CIP (Cleaning-in-place) (20) pour nettoyer la souffleuse (1),
**caractérisée en ce que** les blocs de commande (5) sont conçus pour pouvoir être intégrés au nettoyage CIP.

2. Souffleuse selon la revendication 1, **caractérisée en ce que** les soupapes (6, 7) sont à actionner par voie pneumatique.

3. Souffleuse selon l'une au moins des revendications précédentes, **caractérisée en ce qu'**il est prévu sur les soupapes (6, 7) des soupapes d'arrêt CIP (48) pour couper les conduites de commande pneumatiques (47) des soupapes (6, 7).

4. Souffleuse selon l'une au moins des revendications précédentes, **caractérisée en ce que** les soupapes (6, 7) comprennent des joints d'étanchéité (45, 46) qui résistent aux agents de nettoyage CIP désinfectants, en particulier au peroxyde d'hydrogène, à l'acide peracétique, à l'alcool et à la lessive de savon.

5. Souffleuse selon l'une au moins des revendications précédentes, **caractérisée en ce que** le système de nettoyage CIP (20) comprend :
- une unité de préparation (22) pour remettre en circulation et préparer un produit de nettoyage CIP (24) ;
- une conduite d'amenée CIP (26) pour le produit de nettoyage (24), qui peut être reliée à une conduite d'amenée d'air de soufflage (12) pour les soupapes (6, 7) ; et
- des capots étanches CIP (32) aptes à être posés/enlevés, pour recevoir le produit de nettoyage (24) au niveau des stations de soufflage (2), les capots étanches CIP (32) étant reliés à une conduite de remise en circulation (30) pour ramener le produit de nettoyage (24) dans l'unité de traitement (22).

6. Souffleuse selon la revendication 5, **caractérisée par** ailleurs par un répartiteur d'agent (15) pour répartir l'air de soufflage (9) sur les stations de soufflage (2) et pour recueillir et introduire dans la conduite de remise en circulation CIP (30) le produit de nettoyage (24) reçu.

7. Souffleuse selon la revendication 5 ou 6, **caractérisée en ce qu'**il est prévu dans la conduite d'amenée CIP (26) une soupape d'arrêt principale CIP (28).

8. Souffleuse selon l'une au moins des revendications 5 à 7, **caractérisée en ce qu'**il est prévu dans la conduite d'amenée d'air de soufflage (12) une soupape d'arrêt d'air de soufflage (13).

9. Procédé pour le nettoyage CIP de la souffleuse (1) selon la revendication 1, comprenant l'étape suivante :
a) passage d'un produit de nettoyage CIP (24) par les blocs de commande (5) des stations de soufflage (2), y compris les soupapes (6, 7) et les buses de soufflage (17).

10. Procédé selon la revendication 9, comprenant par ailleurs l'étape suivante :
b) coupure de conduites de commande pneumatiques (47) des soupapes (6, 7), l'étape b) étant exécutée avant l'étape a).

11. Procédé selon la revendication 9 ou 10, comprenant par ailleurs l'étape suivante :
c) liaison d'une conduite d'amenée CIP (26) pour le produit de nettoyage (24) avec au moins une conduite d'amenée d'air de soufflage (12) pour les soupapes (6, 7), l'étape c) étant exécutée avant l'étape a).

12. Procédé selon la revendication 11, comprenant par ailleurs l'étape suivante :
d) coupure d'une section située côté compresseur (12a) de la conduite d'amenée d'air de soufflage (12), l'étape d) étant exécutée avant l'étape a).

13. Procédé selon l'une des revendications 9 à 12, comprenant par ailleurs l'étape suivante :
e) réception du produit de nettoyage (24) qui a traversé les blocs de commande (5), et remise en circulation du produit de nettoyage (24) dans un circuit de produit de nettoyage CIP.

14. Procédé selon la revendication 13, comprenant par ailleurs l'étape suivante :
f) traitement du produit de nettoyage (24) remis en circulation, et amenée dans le circuit de produit de nettoyage CIP du produit de nettoyage (24) préparé.

15. Procédé selon l'une des revendications 9 à 14, étant précisé qu'un produit de nettoyage désinfectant traverse les blocs de commande (5), en particulier du peroxyde d'hydrogène, de l'acide peracétique, de l'alcool ou de la lessive de savon.
